# NOUVEAU FASCICULE DE BREVET EUROPEEN

(11) **EP 0 728 021 B2**
(45) Date de publication et mention de la décision concernant l'opposition: **05.03.2008**
(45) Mention de la délivrance du brevet: 06.08.2003
(21) Numéro de dépôt: 95925983.9
(22) Date de dépôt: 08.08.1995
(51) Int. Cl.: A61L 9/04, C11D 3/50

(54) **DISPOSITIF PARFUMANT POUR LE PARFUMAGE ET L'ASSAINISSEMENT D'AIR AMBIANT**
PARFÜMIERUNGSVORRICHTUNG ZUM PARFÜMIEREN UND SANIEREN VON UMGEBUNGSLUFT
PERFUMING DEVICE FOR PERFUMING AND SANITIZING AMBIENT AIR

(30) Priorité: 19.08.1994 CH 256194
(43) Date de publication de la demande: 28.08.1996
(60) Demande divisionnaire: 03005294.8
(73) Titulaire: Reckitt Benckiser (UK) Limited, Slough, Berkshire SL1 3UH (GB)
(72) Inventeur: O'LEARY, Nicholas, Slough Berkshire SL3 9HS (GB)
(74) Mandataire: Bowers, Craig Malcolm
(86) Numéro de dépôt international: PCT/IB1995/000621
(87) Numéro de publication internationale: WO 1996/005870

(56) Documents cités:
- DE-A- 3 015 460
- FR-A- 2 302 749
- FR-A- 2 455 068
- US-A- 4 362 841
- US-A- 4 497 663

## Description

### Domaine technique

La présente invention a trait au domaine de la parfumerie et plus particulièrement elle a pour objet un dispositif pour le parfumage, la désodorisation et l'assainissement d'air ambiant ou d'enceintes fermées.

### Technique antérieure

L'emploi de dispositifs divers pour l'assainissement d'air ambiant a augmenté considérablement au cours des dernières années. Des désodorisants d'air ambiant par exemple sont désormais d'un usage courant dans tout ménage où ils sont utilisés tout aussi bien pour masquer les mauvaises odeurs que pour conférer des fragrances d'ambiance. Il en va de même dans les lieux publics, les bureaux ou les voitures par exemple.

Les solutions appliquées pour réaliser de tels dispositifs sont des plus variées. A titre indicatif on peut mentionner ici les systèmes basés sur la diffusion rapide d'agents assainissants, diffusion promue par l'action de diffuseurs de type "spray", aérosols ou mécaniques. Egalement connus sont les dispositifs solides constitués par des éléments imprégnés d'ingrédients assainissants actifs, lesquels éléments sont constitués par des gels, tels les gels d'agar-agar ou de stéarate de sodium, voire par des blocs de résine synthétique ou de matériaux minéraux, le plâtre par exemple ou la silice. L'état de la technique est également riche en exemples dans lesquels les dispositifs désodorisants sont constitués par des éléments d'emballage plastique renfermant les ingrédients actifs sous forme liquide, la diffusion des vapeurs assainissantes pouvant s'effectuer au travers des parois senti-perméables polymériques. Enfin, bien connus sont également les dispositifs dans lesquels la diffusion a lieu par l'entremise d'une mèche mise en contact, par l'une de ses extrémités, avec le liquide assainissant.

A l'expérience, il apparaît qu'aucun des systèmes existants ne peut satisfaire l'ensemble des critères de fonctionnalité et d'esthétique requis pour un emploi généralisé, certains dispositifs pouvant mieux s'adapter au parfumage intermittant de petits ou grands espaces, d'autres servant au contraire à l'assainissement continu.

Parmi les systèmes simples à l'efficacité éprouvée figurent ceux basés sur la diffusion d'agents assainissants dans lesquels le support est constitué par un matériau gélifié. Il s'agit de dispositifs qui utilisent en général les propriétés gélifiantes des carragénanes ou des alginates.

Le document FR-A-2 302 749 décrit la préparation d'un dispositif pour le parfumage, qui comprend la polymérisations de monomères en présence d'une base parfumante.

### Exposé de l'invention

Nous avons maintenant découvert qu'on pouvait obtenir des gels, pour emploi en tant que support dans des dispositifs pour parfumer, désodoriser ou assainir, par réaction, en présence d'une base parfumante, d'une substance polymérique avec un agent de lien réticulant.

Comme il apparaitra plus clairement à la lecture des exemples spécifiques donnés par la suite, les dispositifs ainsi préparés offrent plusieurs avantages par rapport aux articles de l'art antérieur. A cet égard on peut citer le fait que le support, une fois formé, apparaît comme un matériau transparent rigide et sec, pouvant renfermer une proportion élevée, jusqu'à 90% ou plus, de base parfumante. Cette dernière caractéristique est particulièrement importante car les dispositifs de l'invention peuvent ainsi être utilisés sous forme d'objets discrets de dimension réduite mais malgré tout efficaces. C'est ainsi que par exemple de tels dispositifs peuvent trouver un emploi intéressant pour le parfumage de petits espaces clos, tels que voitures, salles de bains, cabinets de toilette entre autres. Du fait de leur transparence, ils peuvent être façonnés dans des formes variées et esthétiquement plaisantes. Ce qui plus est, leur fabrication est fort simple car, une fois homogénéisé, le mélange polymérique de réaction contenant la base parfumante se présente sous forme d'un liquide qui peut être aisément versé dans des moules de forme appropriée et variée à souhait.

Enfin, des objets solides peuvent être façonnés en réduisant quelque peu la proportion de la base parfumante -par exemple à environ 70% en poids par rapport au poids du mélange polymérique - et en ajoutant au mélange de réaction des agents de remplissage tel du plâtre, de la sciure, de la poudre métallique ou autres matériaux similaires.

Nous avons découvert que certains polymères fonctionnalisés pouvaient être réticulés in situ, en présence d'une base parfumante, pour former des gels qui présentent les caractéristiques utiles mentionnées plus haut.

La présente invention concerne un procédé pour la préparation d'un dispositif pour le parfumage, la désodorisation ou l'assainissement de l'air ambiant ou d'enceintes fermées, lequel dispositif comprend un élément gélifié, lequel est préparé par mélange d'un polymère, d'un agent réticulant et d'un parfum, d'une base désodorisante ou assainissante ou d'un agent surfactant, en présence ou l'absence d'un solvant, un solvant organique le cas échéant, et par réticulation du polymère avec l'agent réticulant, et dans lequel le polymère est un polymère liquide fonctionnalisé choisi parmi le polybutadiène maléinisé et le polyisoprène maléinisé, ou un copolymère d'éthylène et d'anhydride maléique.

La présente invention concerne également un procédé pour la préparation d'un dispositif pour le parfumage de l'air ambiant ou d'enceintes fermées, lequel dispositif comprend un élément gélifié, lequel est préparé par mélange d'un polymère, d'un agent réticulant et d'une base parfumante, en présence ou l'absence d'un solvant, un solvant organique le cas échéant, et par réticulation du polymère avec l'agent réticulant, dans lequel la base parfumante est présente dans une proportion comprise entre 70 % et 90 % en poids par rapport au poids du mélange constitué par le polymère et l'agent réticulant.

Un des objets de la présente invention consiste en un dispositif pour le parfumage, la désodorisation ou l'assainissement d'air ambiant ou d'enceintes fermées, lequel dispositif comprend un élément gélifié résultant du mélange d'un polymère liquide fonctionnalisé choisi parmi le polybutadiène maléinisé et le polyisoprène maléinisé, ou un copolymère d'éthylène et d'anhydride maléique, d'un agent réticulant et d'un parfum, d'une base désodorisante ou assainissante ou d'un agent surfactant, en présence ou en l'absence d'un solvant, ledit solvant étant un solvant organique le cas échéant, et de la réticulation du polymère avec l'agent réticulant. Ainsi, la réticulation est effectuée entre polymères ayant une fonctionnalité complémentaire.

L'invention a également pour objet un dispositif pour le parfumage d'air ambiant ou d'enceintes fermées, lequel dispositif comprend un élément gélifié résultant du mélange d'un polymère, d'un agent réticulant polymérique et d'une base parfumante, en présence ou en l'absence d'un solvant, ledit solvant étant un solvant organique le cas échéant, et de la réticulation du polymère avec l'agent réticulant polymérique de sorte que la réticulation est effectuée entre polymères ayant une fonctionnalité complémentaire, et dans lequel la base parfumante est présente dans une proportion comprise entre 70 % et 90 % en poids par rapport au poids du mélange constitué par le polymère et l'agent réticulant.

L'invention a en outre pour objet un dispositif pour le parfumage, la désodorisation ou l'assainissement d'air ambiant ou d'enceintes fermées, lequel dispositif comprend un élément gélifié résultant du mélange d'un polymère, d'un agent réticulant polymérique et d'un parfum, d'une base désodorisante ou assainissante ou d'un agent surfactant, en présence ou en l'absence de solvant, ledit solvant étant un solvant organique le cas échéant, et de la réticulation du polymère avec l'agent réticulant polymérique de sorte que la réticulation est effectuée entre polymères ayant une fonctionnalité complémentaire, et dans lequel le polymère est un polymère liquide fonctionnalisé choisi, parmi le polybutadiène maléinisé et le polyisoprène maléinisé, ou un copolymère d'éthylène et d'anhydride maléique.

Selon un autre aspect, l'invention concerne également un procédé pour le parfumage, la désodorisation ou l'assainissement d'air ambiant ou d'enceintes fermées, caractérisé en ce qu'on expose à l'action de l'air environnant, dans une pièce ou enceinte fermée, un dispositif selon l'invention.

Parmi les polymères fonctionnalisés, dont l'emploi est préférentiel selon l'invention, figurent les polymères miscibles aux ingrédients courants de la parfumerie, lesquels polymères possèdent un ou plusieurs groupes fonctionnels. Il en va de même pour l'agent de lien réticulant qui doit posséder un ou plusieurs groupes fonctionnels complémentaires. Le mélange de ces deux éléments, en présence de la base parfumante, produit une réaction dont l'effet résulte en la formation d'un réseau tridimensionnel renfermant la base parfumante.

Les groupes fonctionnels ainsi que les groupes fonctionnels complémentaires peuvent être respectivement des groupes dérivés d'acide carboxylique, anhydride, chlorure d'acide, amine ou alcool. De préférence on choisira des polymères liquides possédant des fonctions dérivées d'acide carboxylique, anhydride ou chlorure d'acide et des agents de lien réticulants ayant une fonction amine ou alcool, l'inverse pouvant également s'appliquer. Les groupes fonctionnels sur le polymère ou l'agent de lien réticulant peuvent être mono- ou poly-fonctionnds. Afin d'obtenir un réseau tridimensionnel il faut toutefois la présence d'au moins deux groupes fonctionnels par molécule, tant pour le polymère liquide que pour l'agent réticulant.

Par "polymère liquide fonctionnalisé" on entend ici un matériau qui est liquide à température ambiante et qui possède une viscosité inférieure à 5000 poise à 25°C, de préférence de l'ordre d'environ 250 à 1000 poise. Comme indiqué plus haut, tant le polymère liquide que l'agent réticulant doivent être solubles dans la base parfumante. Une telle solubilité peut être obtenue par dissolution directe dans la base parfumante ou par adjonction d'un solvant spécifique et approprié. En pratique, il est apparu qu'avec l'emploi de proportions élevées de base parfumante, telles que celles utilisées selon les modes préférentiels de l'invention, un tel ajout est superflu.

Le polymère peut être choisi parmi les nombreux polymères capables d'être fonctionnalisés. De préférence, on choisira une polyoléfine et plus particulièrement un polymère d'une mono- ou di-oléfine contenant, avant fonctionnalisation, au moins un ou, de préférence, plusieurs groupes vinyliques.

### Manières de réaliser l'invention

Selon un mode d'exécution préférentiel de l'invention, on utilise des polymères liquides fonctionnalisés. Préférentiellement on utilise des polymères synthétiques dérivés du butadiène, isoprène ou chloroprène. De préférence on emploie du polybutadiène maléinisé à PM de 5'000-20'000 ou du polyisoprène maléinisé à PM de 200'000-500'000. De tels polymères sont disponibles sur le marché. Il s'agit par exemple de matériaux mentionnés dans la demande de brevet européenne publiée sous le N° EP-A-023 084. A titre d'exemple on peut citer le produit connu sous le nom de "Lithene" [origine: Revertex Limited]. Parmi les différentes qualités de Lithene disponibles, de bons résultats ont été obtenus par l'emploi de "Lithene N4-9000 10MA" [origine : Revertex Ltd.]; 9000 étant le PM du polybutadiène avant maléinisation, tandis que 10MA indique le degré de maléinisation - dans ce cas 10 parties d'anhydride maléique pour 100 parties de polybutadiène (= environ 9,1%)-.

La demande de brevet européenne dtée plus haut donne également des indications quant au choix des agents de lien réticulants. Le critère discriminant dans ce choix est représenté par la solubilité d'un tel agent dans le milieu réactionnel, notamment vis-à-vis des composants de la base parfumante. Comme suggéré dans la demande citée, parmi les agents qui obéissent à un tel critère figure le dihydroxy polybutadiène. La préférence cependant est donnée à des dérivés d'amines primaires éthoxylées. Parmi ces dernières, il convient tout particulièrement d'utiliser une oléylamine possédant 2 unités d'oxyde d'éthylène par molécule. D'autres agents de lien réticulants peuvent être choisis parmi le groupe d'alkylpropyldiamines à chaîne aliphatique supérieure éthoxylée ou propoxylée (par exemple le "Dicrodamet" ; origine : Croda Chemicals Ltd.), la diéthanolamine ou encore la diéthylènetriamine.

Egalement utiles se sont révélés des agents de lien réticulants constitués par des polyoxyalkylènediamines. Plus particulièrement, l'emploi de Jeffamine D-400, Jeffamine EDR-148 et Jeffamine D-2000 se montre très avantageux (Jeffamine est une marque enregistrée de Huntsman Corp.).

Egalement utiles selon l'invention sont des cocoamines ayant 5 unités d'oxyde d'éthylène par molécule. Il s'agit de produits commerciaux connus sous le nom de "Crodamet" [origine : Croda Chemicals Ltd].

Le polymère et l'agent de lien réticulant sont mélangés dans un rapport molaire d'une valeur comprise entre environ 3:1 et 0,5:1, de préférence de 1:1, basé sur le rapport molaire des groupes fonctionnels présents.

L'élément gélifié selon l'invention peut également résulter du mélange de deux polymères possédant des fonctionnalités complémentaires. Bien entendu, les deux polymères doivent être solubles dans la base parfumante ou assainissante choisie. A titre d'exemple, on peut citer à cet effet le polybutadiène à fonction hydroxylée, tel le HFPB (origine : Revertex Ltd) qui gélifie en mélange avec le polybutadiène maléinisé. Parfois, l'emploi de catalyseurs spécifiques permettent un meilleur contrôle de la gélification et, à cet effet, on utilise des amines tertiaires (ex.: DAMA 1010 ; origine : Albemarle SA). Des mélange de Hycar CTBN 1300x21 (origine: B.F. Goodrich) et polybutadiène maléinisé conviennent également parfaitement.

Selon une variante du dispositif de l'invention, à titre de polymère on peut employer, au lieu d'un polymère liquide fonctionnalisé, un copolymère. Parmi les copolymères utilisés, il convient de citer particulièrement l'EMA, ou copolymère d'éthylène et anhydride maléique, substance qui se présente sous forme d'une poudre homogène fluide.

A titre de base parfumante on utilisera dans le dispositif de l'invention toute composition d'usage courant en parfumerie. Il s'agit dans ce cas de substances chmiques à l'état isolé plus fréquemment cependant de mélanges plus ou moins complexes d'ingrédients liquides volatils soit d'origine synthétique que naturelle. La nature de ces ingrédients peut être trouvée dans les ouvrages spécialisés de la parfumerie, par exemple S. Arctander (Perfume and Flavor Chemicals, Montclair N.J., USA, 1969).

Préférentiellement la base parfumante est présente dans une proportion comprise entre environ 70 et 90% en poids par rapport au poids du mélange constitué par le polymère et l'agent réticulant.

Comme indiqué plus haut, un des éléments caractérisant le polymère ainsi que l'agent de lien réticulant, utiles pour la préparation du dispositif de l'invention, est leur solubilité dans le base parfumante, désodorisante ou assainissante choisie. Il est entendu que tant le polymère liquide que l'agent de lien réticulant peuvent être, avant réticulation, dissous dans des solvants organique appropriés. Toutefois une telle opération se révèle susperflue dans la plupart des cas, la base active, particulièrement la base parfumante, étant suffisante pour les dissoudre rendant par conséquent inutile l'ajout d'un solvant supplémentaire.

Quoique dans ce qui précède, il a été fait mention plus particulièrement de l'effet parfumant exercé par les dispositifs selon l'invention, les mêmes principes s'appliquent à la fabrication de dispositifs analogues destinés à diffuser des vapeurs désodorisantes ou assainissantes, la base parfumante étant dans ce cas remplacée par une base désodorisante, bactéricide, insecticide, répellante ou encore attractante. Par le terme "assainissant" nous nous référons en effet ici non seulement à des substances pouvant augmenter le degré de plaisance de l'air environnant l'observateur mais également à des substances pouvant exercer un effet attractant ou répellant vis-à-vis de certaines espèces d'insectes, par exemple vis-à-vis de la mouche domestique ou des moustiques, ou encore bactéricide.

L'invention est illustrée de manière plus détaillée par les exemples suivants dans lesquels les abréviations ont le sense usuel dans l'art et les températures sont indiquées en degrés centigrades.

### Exemple 1

Dans un récipient approprié on a placé 2,23 g de Lithene N4-9000 10MA auxquels on a ajouté 10,28 g d'une base parfumante (Splash 115.032 BGE ; origine: Firmenich SA, Genève, Suisse) et le mélange a été brassé manuellement. Puis on a ajouté sous agitation 0,34 g de Crodamet 02. Après environ 10 min à température ambiante l'huile polymérique s'était gélifiée renfermant la base parfumante. La gélification se complète au bout d'une demi heure.

### Exemple 2

2,56 g de Lithene N4-9000 10MA ont été brassés manuellement dans un récipient approprié avec 12,43 g d'une base parfumante (Splash 115.032 BGE ; origine : Firmenich SA, Genève, Suisse), puis 0,55 g de Crodamet C5 ont été ajoutés sous agitation. Après environ 2 heures à température ordinaire, l'huile s'était gélifiée formant un bloc rigide sec. La rigidité du produit cependant n'était pas aussi prononcée que celle du produit résultant décrit dans l'exemple 1 et ce même après 24 heures.

### Exemple 3

2,29 g de Lithene N4-9000 10MA ont été brassés manuellement dans un récipient approprié avec 14,95 g d'une base parfumante (Splash 115.032 BGE ; origine: Firmenich SA, Genève, Suisse), puis 1,98 g de Crodamet 02 ont été ajoutés sous agitation. Après environ 20 min à température ambiante le mélange huileux se gélifie. La prise en masse se complète au bout de 50 min. Le produit résultant était plus mou que celui préparé à l'exemple 2 et dont le contenu en base parfumante était de 80% en poids.

### Exemple 4

1,44g de Lithene N4-9000 10MA ont été brassés manuellement dans un bêcher avec 14,93 g d'une base parfumante (Splash 115.032 BGE; origine: Fmnenich SA, Genève, Suisse), puis 0,22g de Crodamet 02 ont été ajoutés sous agitation. Après 40 min le mélange se gélifie. La prise en masse se complète au bout de 3 heures. Le produit résultant, qui contient 90% en poids de base parfumante, était plus mou que celui obtenu conformément aux exemples 2 et 3, contenant respectivement 80 et 85% en poids de base parfumante.

### Exemple 5

2,50 g de Lithene N4-9000 10MA ont été brassés manuellement dans un récipient approprié avec 11,60 g d'une base parfumante (Brissago 144.034; origine: Firmenich SA, Genève, Suisse), puis 0,4 g de Crodamet 02 ont été ajoutés sous brassage manuel. Le mélange huileux résultant est ensuite versé dans des moules de type Barex. Après 15 min. à température ambiante l'huile s'est solidifiée en une masse gélifiée caoutchouteuse sèche.

### Exemples 6 - 8

En procédant comme indiqué à l'exemple précédent mais en remplaçant la base parfumante Brissago par des proportions identiques de respectivement Centifoline 144.036, Citronia 144.037 et Tristan 431.756 (origine : Firmenich SA, Genève, Suisse) on a obtenu des gels parfumants de bonne qualité après un temps de gélification compris entre 10 et 25 minutes.

### Exemple 9

2,54 g de Lithene N4-9000 10MA ont été mélangés avec 6,23 g d'une base parfumante (Terminator 109365B ; origine : Firmenich SA, Genève, Suisse) et le mélange a été brassé manuellement. 0,13 g de Crodamet 02 (rapport Lithene/Crodamet 3:1 environ) ont été ajoutés sous brassage. L'huile résultante s'est solidifiée sous forme de gel au bout de 15 min à température ambiante.

### Exemple 10

En opérant comme indiqué à l'exemple précédent mais en utilisant un rapport molaire de Lithene/Crodamet de 5:1 au lieu de 3:1 comme indiqué à l'exemple précédent, on a obtenu un gel collant dont les caractéristiques indiquaient un certain manque de rigidité.

### Exemple 11

1,87 g de Lithene N4-9000 10MA ont été mélangés avec 5,69 g d'une base parfumante (Terminator 109365B; origine : Firmenich SA, Genève, Suisse) puis 0,57 g de Crodamet 02 y ont été ajoutés avec brassage manuel. Après 20 min environ à température ambiante le mélange huileux s'est solidifié sous forme de gel.

### Exemple 12

2 G environ de Lithene N4-9000 10MA ont été versés dans un récipient approprié et mélangés avec la quantité requise de base parfumante (Honeysuckle 150061 ; origine : Firmenich SA) jusqu'à dissolution complète. Les agents de lien réticulants ont été pré-mélangés et ajoutés en malaxant à la solution de polymère-base parfumante, puis le mélange résultant a été versé dans des moules et laissé à repos à température ambiante jusqu'à gélification complète.

| | | | | |
|---|---|---|---|---|
| % w/w Honeysuckle 150061 | 80,00 | 80,00 | 80,00 | 80,00 |
| % w/w Lithène N4/9000 10MA | 17,14 | 17,49 | 17,85 | 18,24 |
| % w/w Jeffamine D-400 | 2,86 | 2,27 | 1,66 | 1,01 |
| % w/w Jeffamine EDR-148 | - | 0,24 | 0,49 | 0,75 |
| Temps de gélification (min) | 59 | 33 | 21 | 7 |

### Exemple 13

2,13 G de Lithene N4-9000 10MA ont été versés dans un récipient approprié et mélangés avec 9,94 g de base parfumante (Beach 68536; origine : Firmenich SA). Après dissolution complète, on a ajouté sous agitation 0,35 g de Jeffaminc D-400.
5 G du mélange (ce qui correspond à 4 g de base parfumante) ont été versés dans une poche thermoformée (28x46 mm) et laissée gélifier à température ambiante. Laissé à l'air, le dispositif perd 86% du poids original de la base parfumante en l'espace de 43 jours.
Des résultats analogues ont été obtenus en utilisant la base parfumante Fresh Bouquet 433213 (origine : Firmenich SA) employée dans le gel à raison de 80% en poids.

### Exemple 14

1,55 G de Lithene N4-9000 10MA ont été versés dans un récipient approprié et mélangés avec 3,82 g d'une base parfumante (Lavande de Provence 150060; origine: Firmenich SA) jusqu'à dissolution complète.
Dans un récipient séparé, on a mélangé 1,87 g de Hycar CTBN 1300x21 (origine : B. F. Goodrich) avec 4.13 g de la même base parfumante que ci-dessus jusqu'à dissolution complète, puis 5,37 g de cette solution ont été ajoutés à la solution parfumée du polymère obtenu précédemment.
Un gel sec, rigide mais trouble se forme rapidement à température ambiante.

### Exemple 15

17,14 G de Lithene N4-9000 10MA ont été ajoutés à un mélange constitué par 40 g d'un surfactant liquide et 40 g de terpènes orange et le tout a été mélangé manuellement jusqu'à dissolution complète.
2,86 G de Jeffamine D-400 ont été additionnés au mélange sous agitation. En moins de 2 min, à température ambiante, la masse se solidifie sous forme de gel.
La base surfactante employée était :

| | | |
|---|---|---|
| 15.1 | Marlipal 24/70 | (origine : Hüls) |
| 15.2 | Marlipal O13/70 | (origine : Hüls) |
| 15.3 | Lutensol ON70 | (origine : BASF) |

## Revendications

1. Procédé pour la préparation d'un dispositif pour le parfumage, la désodorisation ou l'assainissement de l'air ambiant ou d'enceintes fermées, lequel dispositif comprend un élément gélifié, lequel est préparé par mélange d'un polymère, d'un agent réticulant et d'un parfum, d'une base désodorisante ou assainissante, ou d'un agent surfactant, en présence ou l'absence d'un solvant, un solvant organique le cas échéant, et par réticulation du polymère avec l'agent réticulant, et dans lequel le polymère est un polymère liquide fonctionnalisé choisi parmi le polybutadiène maléinisé et le polyisoprène maléinisé, ou un copolymère d'éthylène et d'anhydride maléique.

2. Procédé pour la préparation d'un dispositif pour le parfumage de l'air ambiant ou d'enceintes fermées, lequel dispositif comprend un élément gélifié, lequel est préparé par mélange d'un polymère, d'un agent réticulant et d'une base parfumante, en présence ou l'absence d'un solvant, un solvant organique le cas échéant, et par réticulation du polymère avec l'agent réticulant, dans lequel la base parfumante est présente dans une proportion comprise entre 70 % et 90 % en poids par rapport au poids du mélange constitué par le polymère et l'agent réticulant.

3. Procédé selon la revendication 2, **caractérisé en ce que** le polymère est un polymère liquide fonctionnalisé choisi parmi le polybutadiène maléinisé et le polyisoprène maléinisé, ou un copolymère d'éthylène et d'anhydride maléique.

4. Procédé selon la revendication 1 ou 3, **caractérisé en ce que** le polymère liquide fonctionnalisé est choisi parmi le polybutadiène maléinisé à PM de 5'000 - 20'000 et le polyisoprène maléinisé à PM de 200'000 - 500'000.

5. Procédé selon la revendication 4, **caractérisé en ce que** le polymère liquide fonctionnalisé est le polybutadiène maléinisé à PM de 5'000 - 20'000.

6. Procédé selon l'une quelconque des revendications 1 ou 3 à 5, **caractérisé en ce que** le polymère liquide fonctionnalisé est choisi parmi le polybutadiène maléinisé et le polyisoprène maléinisé, ou un copolymère d'éthylène et d'anhydride maléique, et l'agent réticulant est composé d'une oléylamine possédant 2 unités d'oxyde d'éthylène par molécule ou d'une cocoamine ayant 5 unités d'oxyde d'éthylène par molécule.

7. Procédé selon l'une quelconque des revendications 1 à 6, effectué en l'absence d'un solvant.

8. Procédé selon l'une quelconque des revendications 1 à 7, **caractérisé en ce que** la réticulation est effectuée entre deux polymères ayant une fonctionnalité complémentaire.

9. Procédé selon la revendication 8, **caractérisé en ce que** la réticulation est effectuée entre un polybutadiène à fonction hydroxylée et un polybutadiène maléinisé.

10. Procédé selon la revendication 9, **caractérisé en ce que** la réticulation est effectuée en présence d'une amine tertiaire.

11. Procédé selon l'une quelconque des revendications précédentes, **caractérisé en ce que** le polymère et l'agent de réticulation sont solubles dans la base parfumante, désodorisante ou assainissante.

12. Procédé selon l'une quelconque des revendications précédentes, **caractérisé en ce que** l'agent réticulant est choisi parmi le dihydroxy polybutadiène, les dérivés d'amines primaires éthoxylées, les alkylpropyldiamines ayant une chaîne aliphatique supérieure éthoxylée ou propoxylée, la diéthanolamine, la diéthylènetriamine, les polyoxyalkylène diamines, et les cocoamines ayant 5 unités d'oxyde d'éthylène par molécule.

13. Procédé selon la revendication 12, **caractérisé en ce que** l'amine primaire éthoxylée est une oléylamine à 2 unités d'oxyde d'éthylène par molécule.

14. Procédé selon l'une quelconque des revendications précédentes, **caractérisé en ce que** le polymère et l'agent réticulant sont présents dans un rapport molaire de 1:1, basé sur le rapport molaire des groupes fonctionnels présents.

15. Dispositif pour le parfumage, la désodorisation ou l'assainissement d'air ambiant ou d'enceintes fermées, lequel dispositif comprend un élément gélifié résultant du mélange d'un polymère liquide fonctionnalisé choisi parmi le polybutadiène maléinisé et le polyisoprène maléinisé, ou un copolymère d'éthylène et d'anhydride maléique, d'un agent réticulant et d'un parfum, d'une base désodorisante ou assainissante ou d'un agent surfactant, en présence ou en l'absence de solvant, ledit solvant étant un solvant organique le cas échéant, et de la réticulation du polymère avec l'agent réticulant.

16. Dispositif pour le parfumage d'air ambiant ou d'enceintes fermées, lequel dispositif comprend un élément gélifié résultant du mélange d'un polymère, d'un agent réticulant polymérique et d'une base parfumante, en présence ou en l'absence d'un solvant, ledit solvant étant un solvant organique le cas échéant, et de la réticulation du polymère avec l'agent réticulant polymérique de sorte que la réticulation est effectuée entre polymères ayant une fonctionnalité complémentaire, et dans lequel la base parfumante est présente dans une proportion comprise entre 70 % et 90 % en poids par rapport au poids du mélange constitué par le polymère et l'agent réticulant.

17. Dispositif pour le parfumage, la désodorisation ou l'assainissement d'air ambiant ou d'enceintes fermées, lequel dispositif comprend un élément gélifié résultant du mélange d'un polymère, d'un agent réticulant polymérique et d'un parfum, d'une base désodorisante ou assainissante ou d'un agent surfactant, en présence ou en l'absence de solvant, ledit solvant étant un solvant organique le cas échéant, et de la réticulation du polymère avec l'agent réticulant polymérique de sorte que la réticulation est effectuée entre polymères ayant une fonctionnalité complémentaire, et dans lequel le polymère est un polymère liquide fonctionnalisé choisi parmi le polybutadiène maléinisé et le polyisoprène maléinisé, ou un copolymère d'éthylène et d'anhydride maléique.

18. Dispositif selon la revendication 17, **caractérisé en ce que** le polymère est un polymère liquide fonctionnalisé choisi parmi le polybutadiène maléinisé et le polyisoprène maléinisé, ou un copolymère d'éthylène et d'anhydride maléique.

19. Dispositif selon la revendication 17 ou 18, **caractérisé en ce que** la réticulation est effectuée entre un polybutadiène à fonction hydroxylée et un polybutadiène maléinisé.

20. Dispositif selon la revendication 19, **caractérisé en ce que** la réticulation est effectuée en présence d'une amine tertiaire.

21. Dispositif selon l'une quelconque des revendications 15 ou 17 à 20, **caractérisé en ce que** le polymère liquide fonctionnalisé est choisi parmi le polybutadiène maléinisé à PM de 5'000 - 20 000 et le polyisoprène maléinisé à PM de 200'000 - 500'000.

22. Dispositif selon la revendication 21, **caractérisé en ce que** le polymère liquide fonctionnalisé est le polybutadiène maléinisé à PM de 5'000 - 20'000.

23. Dispositif selon l'une quelconque des revendications 15 ou 17 à 22, **caractérisé en ce que** le polymère liquide fonctionnalisé est choisi parmi le polybutadiène maléinisé et le polyisoprène maléinisé, ou un copolymère d'éthylène et d'anhydride maléique, et l'agent réticulant est composé d'une oléylamine possédant 2 unités d'oxyde d'éthylène par molécule ou d'une cocoamine ayant 5 unités d'oxyde d'éthylène par molécule.

24. Dispositif selon l'une quelconque des revendications 15 à 23, **caractérisé en ce que** le polymère et l'agent réticulant sont solubles dans la base parfumante désodorisante ou assainissante.

25. Dispositif selon l'une quelconque des revendications 15 à 24, **caractérisé en ce que** l'agent réticulant est choisi parmi le dihydroxy polybutadiène, les dérivés d'amines primaires éthoxylées, les alkylpropyldiamines, ayant une chaîne aliphatique supérieure éthoxylée ou propoxylée, la diéthanolamine, le diéthylènetriamine, les polyoxyalkylène diamines, et les cocoamines ayant 5 unités d'oxyde d'éthylène par molécule.

26. Dispositif selon la revendication 25, **caractérisé en ce que** l'amine primaire éthoxylée est une oléylamine à 2 unités d'oxyde d'éthylène par molécule.

27. Dispositif selon l'une quelconque des revendications 15 à 26, **caractérisé en ce que** le polymère et l'agent réticulant sont présents dans un rapport molaire de 1:1, basé sur le rapport molaire des groupes fonctionnels présents.

28. Procédé pour le parfumage, la désodorisation ou la purification de l'air ambiant ou de pièces fermées, **caractérisé en ce que** le dispositif selon l'une quelconque des revendications 15 à 27 est exposé à l'action de l'air environnant dans une pièce ou enceinte fermée.

## Claims

1. A process for preparing a device for perfuming, deodorizing or sanitizing ambient air or enclosed spaces which comprises a gel element, said gel element being prepared by mixing a polymer, a cross-linking agent and a perfume, deodorizing or sanitizing base or a surfactant in the presence or absence of a solvent, wherein, if a solvent is present, it consists of an organic solvent, and cross-linking the polymer with the cross-linking agent, and wherein the polymer is a functionalized liquid polymer chosen from maleinized polybutadiene and maleinized polyisoprene, or a copolymer of ethylene and maleic anhydrid.

2. A process for preparing a device for perfuming ambient air or enclosed spaces which comprises a gel element, said gel element being prepared by mixing a polymer, a cross-linking agent and a perfume base in the presence or absence of a solvent, wherein, if a solvent is present, it consists of an organic solvent, and cross-linking the polymer with the cross-linking agent, wherein the perfume base is present in a proportion of between 70 % and 90 % by weight with respect to the weight of the mixture composed of the polymer and the cross-linking agent.

3. A process according to claim 2, **characterized in that** the polymer is a functionalized liquid polymer chosen from maleinized polybutadiene and maleinized polyisoprene, or a copolymer of ethylene and maleic anhydrid.

4. A process according to claim 1 or 3, **characterized in that** the functionalized liquid polymer is chosen from maleinized polybutadiene with an MW of 5,000-20,000 and maleinized polyisoprene with an MW of 200,000-500,000.

5. A process according to claim 4, **characterized in that** the functionalized liquid polymer is the maleinized polybutadiene with an MW of 5,000-20,000.

6. A process according to any one of claims 1 or 3 to 5, **characterized in that** the functionalized liquid polymer is chosen from maleinized polybutadiene and maleinized polyisoprene, or a copolymer of ethylene and maleic anhydrid, and the cross-linking agent is composed either of an oleylamine having 2 ethylene oxide units per molecule or of a cocoamine having 5 ethylene oxide units per molecule.

7. A process according to any one of claims 1 to 6, carried out in the absence of a solvent.

8. A process according to any one of claims 1 to 7, **characterized in that** the cross-linking is carried out between two polymers having a complementary functionality.

9. A process according to claim 8, **characterized in that** the cross-linking is carried out between a hydroxylated polybutadiene and a maleinized polybutadiene.

10. A process according to claim 9, **characterized in that** the cross-linking is carried out in the presence of a tertiary amine.

11. A process according to any one of preceding claims, **characterized in that** the polymer and the cross-linking agent are soluble in the perfuming, deodorizing or sanitizing base.

12. A process according to any one of preceding claims, **characterized in that** the cross-linking agent is chosen from dihyroxypolybutadiene, ethoxylated primary amine derivatives, alkylpropyldiamines, having an ethoxylated or propoxylated higher aliphatic chain, diethanolamine, diethylenetriamine, polyoxyalkylenediamines, and cocoamines having 5 ethylene oxide units per molecule.

13. A process according to claim 12, **characterized in that** the ethoxylated primary amine is an oleylamine having 2 ethylene oxide units per molecule.

14. A process according to any one of preceding claims, **characterized in that** the polymer and the cross-linking agent are present in a molar ratio of 1:1, based on the molar ratio of the functional groups which are present.

15. A device for perfuming, deodorizing or sanitizing ambient air or enclosed spaces which comprises a gel element resulting from the mixing of a functionalized liquid polymer chosen from maleinized polybutadiene and maleinized polyisoprene, or a copolymer of ethylene and maleic anhydrid, a cross-linking agent and a perfume, deodorizing or sanitizing base or a surfactant, in the presence or absence of a solvent, wherein, if a solvent is present, it consists of an organic solvent, and cross-linking the polymer with the cross-linking agent.

16. A device for perfuming ambient air or enclosed spaces which comprises a gel element resulting from the mixing of a polymer, a polymeric cross-linking agent and a perfume base in the presence or absence of a solvent, wherein, if a solvent is present, it consists of an organic solvent, and cross-linking the polymer with the cross-linking agent, such that the cross-linking is carried out between polymers having a complementary functionality, and wherein the perfume base is present in a proportion of between 70 % and 90 % by weight with respect to the weight of the mixture composed of the polymer and the cross-linking agent.

17. A device for perfuming, deodorizing or sanitizing ambient air or enclosed spaces which comprises a gel element resulting from the mixing of a polymer, a polymeric cross-linking agent and a perfume, deodorizing or sanitizing base or a surfactant, in the presence or absence of a solvent, wherein, if a solvent is present, it consists of an organic solvent, and cross-linking the polymer with the cross-linking agent, such that the cross-linking is carried out between polymers having a complementary functionality, and wherein the polymer is a functionalized liquid polymer chosen from maleinized polybutadiene and maleinized polyisoprene, or a copolymer of ethylene and maleic anhydrid.

18. A device according to claim 17, **characterized in that** the polymer is a functionalized liquid polymer chosen from maleinized polybutadiene or a copolymer of ethylene and maleic anhydrid.

19. A device according to claim 17 or 18, **characterized in that** the cross-linking agent is chosen between a hydroxylated polybutadiene and a maleinized polybutadiene.

20. A device according to claim 19, **characterized in that** the cross-linking is carried out in the presence of a tertiary amine.

21. A device according to any one of claims 15 or 17 to 20, **characterized in that** the functionalized liquid polymer is chosen from maleinized polybutadiene with an MW of 5,000-20,000 and maleinized polyisoprene with an MW of 200,000-500,000.

22. A device according to claim 21, **characterized in that** the functionalized liquid polymer is maleinized polybutadiene with an MW of 5,000-20,000.

23. A device according to any one of claims 15 or 17 to 22, **characterized in that** the functionalized liquid polymer is chosen from maleinized polybutadiene and maleinized polyisoprene, or a copolymer of ethylene and maleic anhydrid, and the cross-linking agent is composed either of an oleylamine having 2 ethylene oxide units per molecule or of a cocoamine having 5 ethylene oxide units per molecule.

24. A device according to any one of claims 15 to 23, **characterized in that** the polymer and the cross-linking agent are soluble in the perfuming, deodorizing or sanitizing base.

25. A device according to any one of claims 15 to 24, **characterized in that** the cross-linking agent is chosen from dihyroxypolybutadiene, ethoxylated primary amine derivatives, alkylpropyldimanes, having an ethoxylated or propoxylated higher aliphatic chain, diethanolamine, diethylenetriamine, polyoxyalkylenediamines, and cocoamines having 5 ethylene oxide units per molecule.

26. A device according to claim 25, **characterized in that** the ethoxylated primary amine is an oleylamine having 3 ethylene oxide units per molecule.

27. A device according to any one of claims 15 to 26, **characterized in that** the polymer and the cross-linking agent are present in a molar ratio of 1:1 based on the molar ratio of the functional groups which are present.

28. A process for the scenting, deodorizing or purifying of ambient air or of closed rooms, **characterized in that** a device according to any one of claims 15 to 27 is exposed to the effect of the surrounding air in a closed room or chamber.

## Patentansprüche

1. Verfahren zur Herstellung einer Vorrichtung zur Parfümierung, Desodorierung oder Verbesserung der Umgebungsluft oder der Luft in geschlossenen Räumen, wobei die Vorrichtung ein geliertes Element umfasst, das durch Vermischen eines Polymers, eines Vernetzungsmittels und eines Parfums, eines desodorierenden oder luftverbessernden Grundbestandteils oder eines oberflächenaktiven Mittels, in Gegenwart oder Abwesenheit eines Lösungsmittels, gegebenenfalls eines organischen Lösungsmittels, und durch Vernetzung des Polymers mit dem Vernetzungsmittel hergestellt wird, und wobei das Polymer ein flüssiges funktionalisiertes Polymer, ausgewählt aus maleinisiertem Polybutadien und maleinisiertem Polyisopren oder einem Ethylen-Maleinsäureanhydrid-Copolymer, ist.

2. Verfahren zur Herstellung einer Vorrichtung zur Parfümierung der Umgebungsluft oder der Luft in geschlossenen Räumen, wobei die Vorrichtung ein geliertes Element umfasst, das durch Vermischen eines Polymers, eines Vernetzungsmittels und eines parfümierenden Grundbestandteils, in Gegenwart oder Abwesenheit eines Lösungsmittels, gegebenenfalls eines organischen Lösungsmittels, und durch Vernetzung des Polymers mit dem Vernetzungsmittel hergestellt wird, wobei der parfümierende Grundbestandteil in einem Anteil zwischen 70 Gewichts-% und 90 Gewichts-%, bezogen auf das Gewicht des Gemisches des Polymers und des Vernetzungsmittels, vorliegt.

3. Verfahren gemäß Anspruch 2, **dadurch gekennzeichnet, dass** das Polymer ein flüssiges funktionalisiertes Polymer, ausgewählt aus maleinisiertem Polybutadien und maleinisiertem Polyisopren oder einem Ethylen-Maleinsäureanhydrid-Copolymer, ist.

4. Verfahren gemäß Anspruch 1 oder 3, **dadurch gekennzeichnet, dass** das flüssige funktionalisierte Polymer ausgewählt ist aus maleinisiertem Polybutadien mit einem Molekulargewicht von 5.000 bis 20.000 und maleinisiertem Polyisopren mit einem Molekulargewicht von 200.000 bis 500.000.

5. Verfahren gemäß Anspruch 4, **dadurch gekennzeichnet, dass** das flüssige funktionalisierte Polymer maleinisiertes Polybutadien mit einem Molekulargewicht von 5.000 bis 20.000 ist.

6. Verfahren gemäß einem der Ansprüche 1 oder 3 bis 5, **dadurch gekennzeichnet, dass** das flüssige funktionalisierte Polymer ausgewählt ist aus maleinisiertem Polybutadien und maleinisiertem Polyisopren oder einem Ethylen-Maleinsäureanhydrid-Copolymer und das Vernetzungsmittel aus einem Oleylamin mit 2 Ethylenoxideinheiten pro Molekül oder einem Kokosamin mit 5 Ethylenoxideinheiten pro Molekül besteht.

7. Verfahren gemäß einem der Ansprüche 1 bis 6, durchgeführt in Abwesenheit eines Lösungsmittels.

8. Verfahren gemäß einem der Ansprüche 1 bis 7, **dadurch gekennzeichnet, dass** die Vernetzung zwischen zwei Polymeren mit einer komplementären Funktionalität erfolgt.

9. Verfahren gemäß Anspruch 8, **dadurch gekennzeichnet, dass** die Vernetzung zwischen einem hydroxyfunktionalisierten Polybutadien und einem maleinisierten Polybutadien erfolgt.

10. Verfahren gemäß Anspruch 9, **dadurch gekennzeichnet, dass** die Vernetzung in Gegenwart eines tertiären Amins erfolgt.

11. Verfahren gemäß einem der vorhergehenden Ansprüche, **dadurch gekennzeichnet, dass** das Polymer und das Vernetzungsmittel in dem parfümierenden, desodorierenden oder luftverbessernden Grundbestandteil löslich sind.

12. Verfahren gemäß einem der vorhergehenden Ansprüche, **dadurch gekennzeichnet, dass** das Vernetzungsmittel ausgewählt ist aus Dihydroxypolybutadien, ethoxylierten Derivaten primärer Amine, Alkylpropyldiaminen mit einer ethoxylierten oder propoxylierten höheraliphatischen Kette, Diethanolamin, Diethylentriamin, Polyoxyalkylendiaminen und Kokosaminen mit 5 Ethylenoxideinheiten pro Molekül.

13. Verfahren gemäß Anspruch 12, **dadurch gekennzeichnet, dass** das primäre ethoxylierte Amin ein Oleylamin mit 2 Ethylenoxideinheiten pro Molekül ist.

14. Verfahren gemäß einem der vorhergehenden Ansprüche, **dadurch gekennzeichnet, dass** das Polymer und das Vernetzungsmittel in einem molaren Verhältnis von 1:1, bezogen auf das molare Verhältnis der vorhandenen funktionellen Gruppen, vorliegen.

15. Vorrichtung zur Parfümierung, Desodorierung oder Verbesserung der Umgebungsluft oder der Luft in geschlossenen Räumen, wobei die Vorrichtung ein geliertes Element umfasst, das durch Vermischen eines flüssigen funktionalisierten Polymers, ausgewählt aus maleinisiertem Polybutadien und maleinisiertem Polyisopren oder einem Ethylen-Maleinsäureanhydrid-Copolymer, eines Vernetzungsmittels und eines Parfüms, eines desodorierenden oder luftverbessernden Grundbestandteils oder eines oberflächenaktiven Mittels, in Gegenwart oder Abwesenheit von Lösungsmittel, das gegebenenfalls ein organisches Lösungsmittel ist, und durch Vernetzung des Polymers mit dem Vernetzungsmittel gebildet wird.

16. Vorrichtung zur Parfümierung der Umgebungsluft oder der Luft in geschlossenen Räumen, wobei die Vorrichtung ein geliertes Element umfasst, das durch Vermischen eines Polymers, eines polymeren Vernetzungsmittels und eines parfümierenden Grundbestandteils, in Gegenwart oder Abwesenheit eines Lösungsmittels, das gegebenenfalls ein organisches Lösungsmittel ist, und durch Vernetzung des Polymers mit dem polymeren Vernetzungsmittel gebildet wird, so dass die Vernetzung zwischen Polymeren mit komplementärer Funktionalität erfolgt, und wobei der parfümierende Grundbestandteil in einem Anteil zwischen 70 Gewichts-% und 90 Gewichts-%, bezogen auf das Gewicht des Gemisches des Polymers und des Vernetzungsmittels, vorliegt.

17. Vorrichtung zur Parfümierung, Desodorierung oder Verbesserung der Umgebungsluft oder der Luft in geschlossenen Räumen, wobei die Vorrichtung ein geliertes Element umfasst, das durch Vermischen eines Polymers, eines polymeren Vernetzungsmittels und eines Parfüms, eines desodorierenden oder luftverbessernden Grundbestandteils oder eines oberflächenaktiven Mittels, in Gegenwart oder Abwesenheit von Lösungsmittel, das gegebenenfalls ein organisches Lösungsmittel ist, und durch Vernetzung des Polymers mit dem polymeren Vernetzungsmittel gebildet wird, so dass die Vernetzung zwischen Polymeren mit komplementärer Funktionalität erfolgt, und wobei das Polymer ein flüssiges funktionalisiertes Polymer, ausgewählt aus maleinisiertem Polybutadien und maleinisiertem Polyisopren oder einem Ethylen-Maleinsäureanhydrid-Copolymer, ist.

18. Vorrichtung gemäß Anspruch 17, **dadurch gekennzeichnet, dass** das Polymer ein flüssiges funktionalisiertes Polymer, ausgewählt aus maleinisiertem Polybutadien und maleinisiertem Polyisopren oder einem Ethylen-Maleinsäureanhydrid-Copolymer, ist.

19. Vorrichtung gemäß Anspruch 17 oder 18, **dadurch gekennzeichnet, dass** die Vernetzung zwischen einem hydroxyfunktionalisierten Polybutadien und einem maleinisierten Polybutadien erfolgt.

20. Vorrichtung gemäß Anspruch 19, **dadurch gekennzeichnet, dass** die Vernetzung in Gegenwart eines tertiären Amins erfolgt.

21. Vorrichtung gemäß einem der Ansprüche 15 oder 17 bis 20, **dadurch gekennzeichnet, dass** das flüssige funktionalisierte Polymer ausgewählt ist aus maleinisiertem Polybutadien mit einem Molekulargewicht von 5.000 bis 20.000 und maleinisiertem Polyisopren mit einem Molekulargewicht von 200.000 bis 500.000.

22. Vorrichtung gemäß Anspruch 21, **dadurch gekennzeichnet, dass** das flüssige funktionalisierte Polymer maleinisiertes Polybutadien mit einem Molekulargewicht von 5.000 bis 20.000 ist.

23. Vorrichtung gemäß einem der Ansprüche 15 oder 17 bis 22, **dadurch gekennzeichnet, dass** das flüssige funktionalisierte Polymer ausgewählt ist aus maleinisiertem Polybutadien und maleinisiertem Polyisopren oder einem Ethylen-Maleinsäureanhydrid-Copolymer, und dass das Vernetzungsmittel aus einem Oleylamin mit 2 Ethylenoxideinheiten pro Molekül oder einem Kokosamin mit 5 Ethylenoxideinheiten pro Molekül besteht.

24. Vorrichtung gemäß einem der Ansprüche 15 bis 23, **dadurch gekennzeichnet, dass** das Polymer und das Vernetzungsmittel in dem parfümierenden, desodorierenden oder luftverbessernden Grundbestandteil löslich sind.

25. Vorrichtung gemäß einem der Ansprüche 15 bis 24, **dadurch gekennzeichnet, dass** das Vernetzungsmittel ausgewählt ist aus Dihydroxypolybutadien, ethoxylierten Derivaten primärer Amine, Alkylpropyldiaminen mit einer ethoxylierten oder propoxylierten höheraliphatischen Kette, Diethanolamin, Diethylentriamin, Polyoxyalkylendiaminen und Kokosaminen mit 5 Ethylenoxideinheiten pro Molekül.

26. Vorrichtung gemäß Anspruch 25, **dadurch gekennzeichnet, dass** das primäre ethoxylierte Amin ein Oleylamin mit 2 Ethylenoxideinheiten pro Molekül ist.

27. Vorrichtung gemäß einem der Ansprüche 15 bis 26, **dadurch gekennzeichnet, dass** das Polymer und das Vernetzungsmittel in einem molaren Verhältnis von 1:1, bezogen auf das molare Verhältnis der vorhandenen funktionellen Gruppen, vorliegen.

28. Verfahren zur Parfümierung, Desodorierung oder Reinigung der Umgebungsluft oder der Luft in geschlossenen Räumen, **dadurch gekennzeichnet, dass** die Vorrichtung gemäß einem der Ansprüche 15 bis 27 der Einwirkung der umgebenden Luft in einem Raum oder einem geschlossenen Raum ausgesetzt wird.
